# EUROPEAN PATENT APPLICATION

(11) **EP 2 600 219 A2**
(43) Date of publication of application: **05.06.2013**
(21) Application number: 12193299.0
(22) Date of filing: 19.11.2012
(51) Int. Cl.: G06F 3/01, A61B 5/0484

(54) **Thought enabled hands-free control of multiple degree-of-freedom systems**

(30) Priority: 30.11.2011 US 201113307580
(71) Applicant: Honeywell International Inc., Morristown, NJ 07962-2245 (US)
(72) Inventor: Mathan, Santosh, Morristown, NJ New Jersey 07962-2245 (US); Conner, Kevin J., Morristown, NJ New Jersey 07962-2245 (US); Erdogmus, Deniz, Morristown, NJ New Jersey 07962-2245 (US)
(74) Representative: Houghton, Mark Phillip

(57) **Abstract**

Systems and methods are provided for controlling a multiple degree-of-freedom system. Plural stimuli are provided to a user, and steady state visual evoked response potential (SSVEP) signals are obtained from the user. The SSVEP signals are processed to generate a system command. Component commands are generated based on the system command, the plurality of components commands causing the multiple degree-of-freedom system to implement the system command.

## Description

### TECHNICAL FIELD

The present invention generally relates to control systems, and more particularly relates to a system and method for thought-enabled, hands-free control of relatively complex, multiple degree-of-freedoms systems.

### BACKGROUND

Human-machine interaction, and most notably human-computer interaction, has become dominated by the graphical user interface (GUI). A typical GUI may implement the so-called "WIMP" (windows, icons, menus, pointing devices) paradigm or, more recently, the touchscreen paradigm. However, it is becoming increasingly evident that these conventional human-computer interface paradigms exhibit significant drawbacks in some operational contexts. For example, in a battlefield context, these paradigms can be difficult to interact with in situations where military personnel may also need to manually manipulate one or more objects, such as a weapon. These human-computer interface paradigms may also be cumbersome and complex in the context of unmanned vehicle operations. Control of these vehicles, which may include both terrestrial and air vehicles, may rely on displays and controls that are distributed over a large area.

In recent years, various hands-free human-computer interface paradigms have been developed. One such paradigm implements an oculo-encephalographic communication system. With this system, electroencephalogram (EEG) sensors are disposed on a person and visual stimuli are presented to the person. The EEG sensors are used to identify a particular visual stimulus at which the person momentarily gazes or pays visual attention to without necessarily directing eye gaze. The visual stimulus being gazed at or attended to may, for example, correspond to a particular command. This command may be used to move a component of a robotic agent. Although this paradigm presents a potential improvement over current GUI paradigms, the systems that have been developed thus far control rather simple, single degree-of-freedom systems and devices, and not more complex, multiple degree-of-freedom systems and devices.

Speech interfaces have been viewed as a solution for hands free control, but they are inappropriate in noisy environments or in environments where spoken communication is a critical component of the task environment. Gesture requires the use of hands, and gaze tracking requires cameras that have limited fields of view, and perform poorly in bright sunlight.

In view of the foregoing, it is clear that the diversity of task contexts in which computing technology is being deployed presents the need for a human-computer interface paradigm that applies flexibly across systems and task contexts. There is also a need for a hands-free paradigm that may be implemented with relatively complex, multiple degree-of-freedom systems and devices. The present invention addresses one or more of these needs.

### BRIEF SUMMARY

In one embodiment, an apparatus for controlling a multiple degree-of-freedom system includes a user interface, a plurality of bioelectric sensors, a processor, and a system controller. The user interface is configured to generate a plurality of stimuli to a user. The bioelectric sensors are each configured to obtain and supply a plurality of steady state visual evoked response potential (SSVEP) signals from the user when the user is receiving the stimuli. The processor is coupled to receive the plurality of SSVEP signals from the EEG sensors and is configured, upon receipt thereof, to determine a system command and supply a system command signal representative thereof. The system controller is coupled to receive the command signal and is configured, upon receipt thereof, to generate a plurality of component commands that cause the multiple degree-of-freedom system to implement the system command.

In another embodiment, a method is provided for controlling a multiple degree-of-freedom system includes displaying, on a visual interface, a plurality of visual stimuli to a user. Steady state visual evoked response potential (SSVEP) signals are obtained from the user when the user is viewing the visual interface. The SSVEP signals are processed to generate a system command. Component commands are generated based on the system command, the plurality of components commands causing the multiple degree-of-freedom system to implement the system command.

In still another embodiment, an apparatus for controlling a multiple degree-of-freedom system includes a visual user interface, a plurality of bioelectric sensors, and a processor. The visual user interface is configured to display a plurality of visual stimuli to a user in accordance with a flickering pattern. The bioelectric sensors are configured to obtain and supply a plurality of steady state visual evoked response potential (SSVEP) signals from the user when the user is viewing the visual interface. The processor is coupled to receive the plurality of SSVEP signals from the bioelectric sensors, and is configured, upon receipt of the SSVEP signals, to determine a system command and supply a system command signal representative thereof. The processor implements a dynamic model of the physical visual system of the user as a communication channel, and a model-based classifier. The dynamic model is representative of the dynamic behavior of the response of the physical visual system to the stimuli, and generates a model-based response to the visual stimuli. The model-based classifier is configured to determine the system command in response to model-based response. The flickering pattern is based on the dynamic model.

Furthermore, other desirable features and characteristics of the thought-enabled hands-free control system and method will become apparent from the subsequent detailed description and the appended claims, taken in conjunction with the accompanying drawings and the preceding background.

### BRIEF DESCRIPTION OF THE DRAWINGS

The present invention will hereinafter be described in conjunction with the following drawing figures, wherein like numerals denote like elements, and wherein:

FIG. 1 depicts a functional block diagram of one embodiment of a thought-enabled hands-free control system for controlling a multiple degree-of-freedom system;

FIG. 2 depicts an example of how visual stimuli may be presented to a user on a visual user interface;

FIG. 3 depicts a simplified representation of a model of a human visual system as a communications channel;

FIG. 4 depicts a functional block diagram of the system of FIG. 1 configured to control an aircraft; and

FIGS. 5 and 6 depict variations of a visual user interface that may be used to implement the system of FIG. 1 to control a robotic system.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

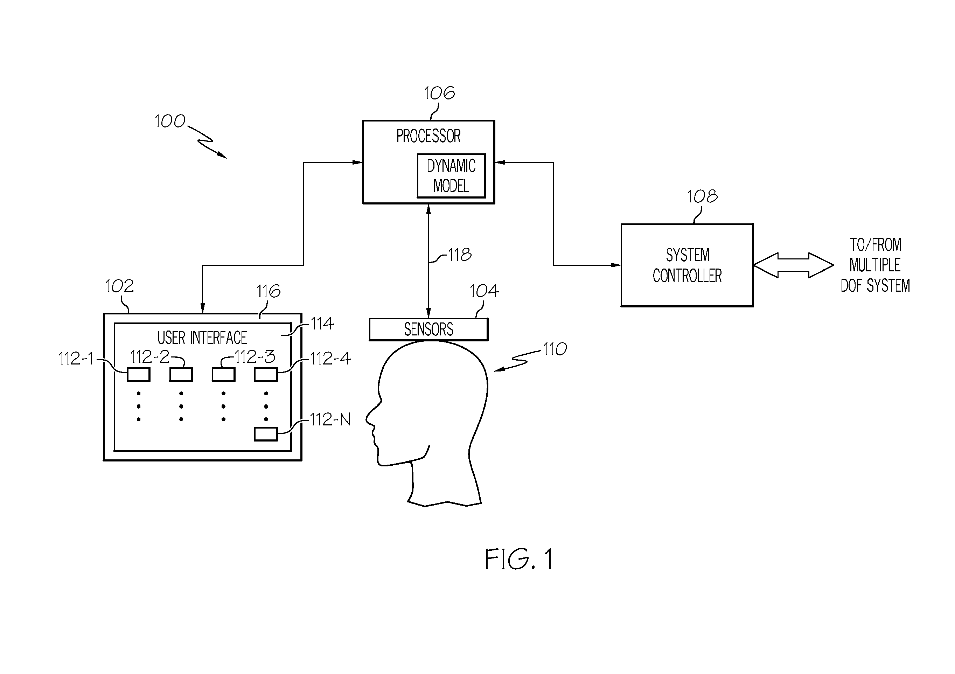

Referring first to FIG. 1, a functional block diagram of one embodiment of a thought-enabled hands-free control system 100 for controlling a multiple degree-of-freedom system is depicted. The system 100 includes a user interface 102, a plurality of bioelectric sensors 104, a processor 106, and a system controller 108. The user interface 102 is configured to supply a plurality of user stimuli 112 (e.g., 112-1, 112-2, 112-3, ... 112-N) to a user 110. The user interface 102 and user stimuli 112 may be variously configured and implemented. For example, the user interface 102 may be a visual interface, a tactile interface, an auditory interface or various combinations thereof. As such, the user stimulus 112 supplied by the user interface may be a visual stimulus, a tactile stimulus, an auditory stimulus, or various combinations thereof. In the depicted embodiment, however, the user interface 102 is a visual user interface and the user stimuli 112 are all implemented as visual stimuli.

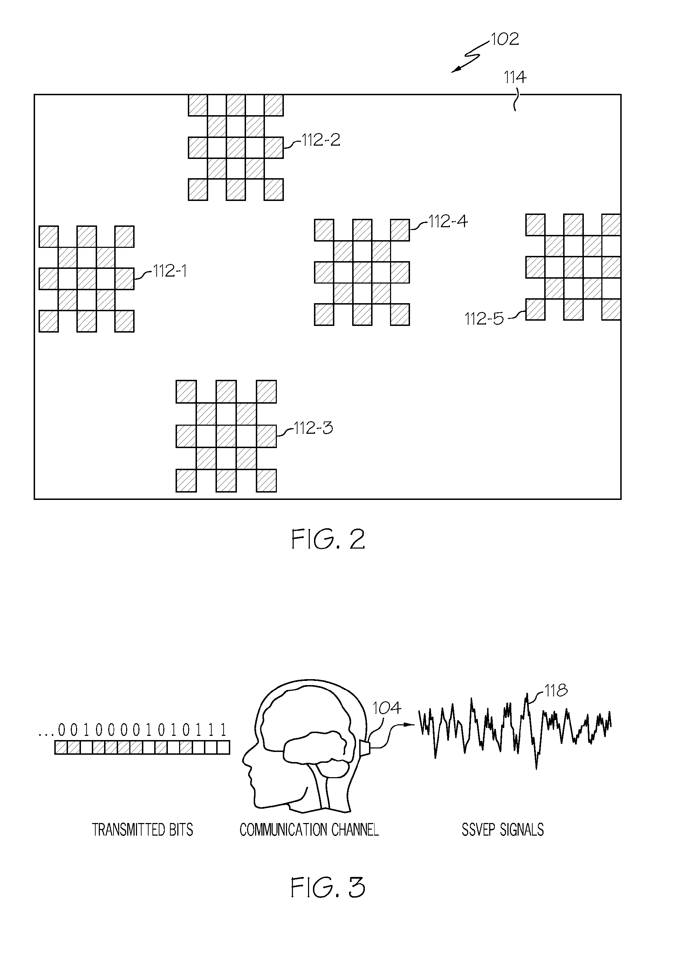

As may be appreciated, the visual user interface 102 may be variously configured and implemented. For example, it may be a conventional display device (e.g., a computer monitor), an array of light sources, such as light emitting diodes (LEDs), that may be variously disposed on the visual user interface 102. The visual stimuli 112 may also be variously implemented. For example, each visual stimulus 112 may be rendered on a display portion 114 of the visual user interface 102 as geometric objects and/or icons, or be implemented using spatially separated lights disposed along a peripheral 116 or other portion of the visual user interface 102, or a combination of both. One example of how visual stimuli 112 may be presented to a user on the visual user interface 102 is depicted in FIG. 2.

No matter how the user interface 102 and user stimuli 112 are specifically implemented, each user stimulus 112 represents a command. As is now generally known, when a user 110 looks at (touches or listens to) a user stimulus 112 of a particular frequency, a cluster of neurons in the rear portion of the user's brain fire synchronously at the same frequency and generate a neural signal that is generally referred to as a steady state visual evoked response potential (SSVEP). An SSVEP is a harmonic neural response to an oscillating visual stimulus, and can be detected using bioelectric sensors. In the depicted embodiment, the sensors are the EEG sensors 104, which are adapted to be disposed on or near the user's head by, for example, embedding the EEG sensors 104 in a helmet or cap. It will be appreciated that EMG (electromyogram) sensors could also be used. The EEG (or EMG) sensors 104 are each configured to obtain and supply a plurality of SSVEP signals 118 from the user 110 when the user is viewing the visual interface 102. The SSVEP signals 118 are supplied to the processor 106.

The processor 106 is coupled to receive the plurality of SSVEP signals 118 from the EEG sensors 104 and is configured, upon receipt of the SSVEP signals 118, to determine a system command, and then supply a system command signal representative of the determined system command. It will be appreciated that the processor 106 may implement this functionality using any one of numerous techniques. For example, the processor 106 may be configured to implement any one of numerous known non-model based classifiers, such as template matching, linear, or quadratic discriminant. In the depicted embodiment, the processor 106 is configured to implement a dynamic model 122, and more specifically, a dynamic model of the visual system (e.g., eyes, retina, visual cortex, etc.) of the user 110. The visual system dynamic model 122 represents the dynamic behavior of the visual system of the user 110 in response to stimuli presented to the user on the visual user interface 102 display (input) and SSVEP signals measured by the EEG sensors 104.

The visual system dynamic model 122 is generated using calibration data obtained from the user 110. The visual system dynamic model 122 may thus be custom fitted to each individual user by using various system identification techniques. Some non-limiting examples of suitable techniques include least-squares regression and maximum likelihood model fitting procedures. The visual system dynamic model 122 may be either linear or non-linear dynamic models. Some non-limiting examples of suitable dynamic models include finite impulse response (FIR) filters, finite-dimensional state linear models, finite-dimensional state nonlinear models, Volterra or Wiener series expansions, and kernel regression machines.

The visual system dynamic model 122 is also used to develop statistical (Bayesian) intent classifiers. The model-based classifiers can be designed to be generative or discriminative. An example of a suitable generative classifier is the minimum Bayesian risk classifier that uses dynamic and statistical models of the SSVEP signals 118 in response to different visual stimuli patterns. An example of a suitable discriminative classifier is a support vector machine that uses, for example, the Fisher kernel obtained from this system model.

One particular advantage of using the dynamic system model 122 is that it may also be thought of as a communication channel through which bits representative of possible commands are transmitted. This concept is illustrated in FIG. 3. As such, information theory and modem coding theory used in digital communications may be employed. In particular, different flickering patterns (or coding schemes) for each visual stimulus 112 may be developed in order to achieve relatively higher, error-free bandwidths that approach the theoretical Shannon capacity of the communication channel. The dynamic system model 122 associated with each user 110 will determine the optimal coding scheme. One particular example of a suitable coding scheme is the phase-shifted m-sequences.

Before proceeding further, it is noted that the processor 106 may also implement various signal processing techniques. These signal processing techniques may vary, and may include one or more of DC drift correction and various signal filtering. The filtering may be used to eliminate noise and various other unwanted signal artifacts due to, for example, noise spikes, muscle artifacts, and eye-blinks.

No matter how the processor 106 specifically implements its functionality, the command signals 118 it generates are supplied to the system controller 108. The system controller 108 and processor 106 together implement a hybrid controller. That is, the system controller 108 is configured, upon receipt of each system command signal 118, to generate a plurality of component commands that cause a multiple degree-of-freedom system (not depicted in FIG. 1) to implement the system command. The system controller 108 is, more specifically, configured to map each received command signal 118 to a plurality of component commands, and to transmit each of the component commands to a different component that comprises the multiple degree-of-freedom system. The different components, in response to the component command each receives, implements the component command, and together these components cause the multiple degree-of-freedom system to implement the system command.

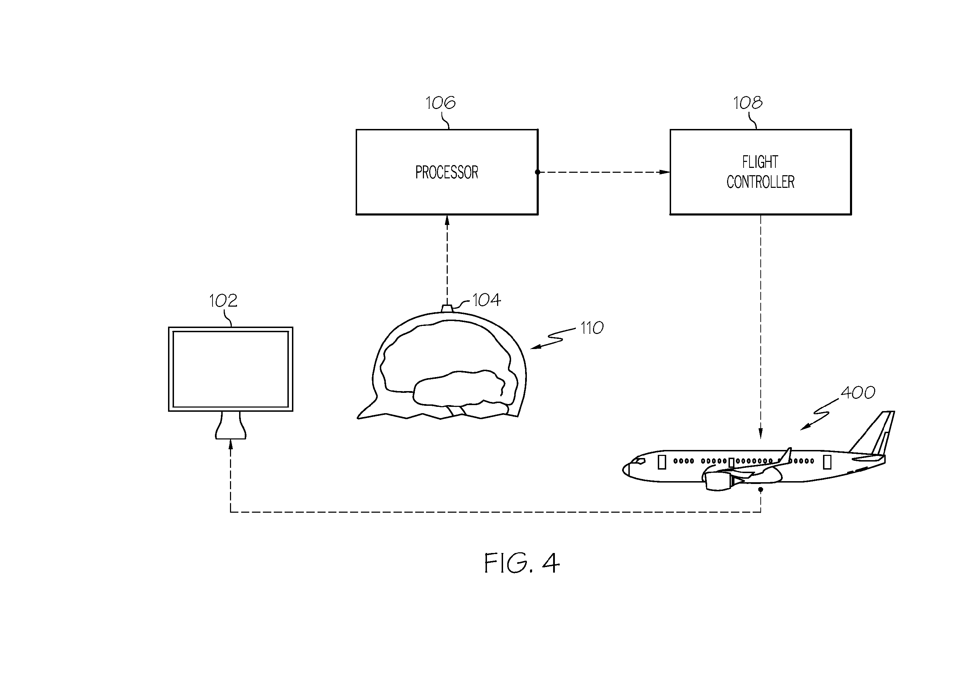

The system 100 depicted in FIG. 1 may be used to control any one of numerous types of multiple degree-of-freedom systems. For example, as depicted in FIG. 4, the system 100 may be used to control an aircraft 300. In such an instance, the system controller 108 of FIG. 1 is implemented as an aircraft flight controller. As is generally known, a flight controller receives aircraft flight control maneuver commands (e.g., roll left, roll right, pitch up, pitch down, etc.) from an user interface. The flight controller 108, in response to the maneuver commands, supplies actuator commands to appropriate flight control surface actuators that in turn cause appropriate flight control surfaces to move to positions that will cause the aircraft 400 to implement the commanded maneuver.

In the context of FIG. 4, the user interface is not a yoke, a cyclical, a control stick, rudder pedals, or any one of numerous other known flight control user interfaces. Rather, the user interface is implemented using the visual user interface 102. In this regard, the visual user interface 102 may be implemented as a device that is separate from the avionics suite, integrated into the avionics suite, or a combination of both. In one particular embodiment, the visual user interface 102 is implemented into an augmented reality display, such as a head-up display (HUD).

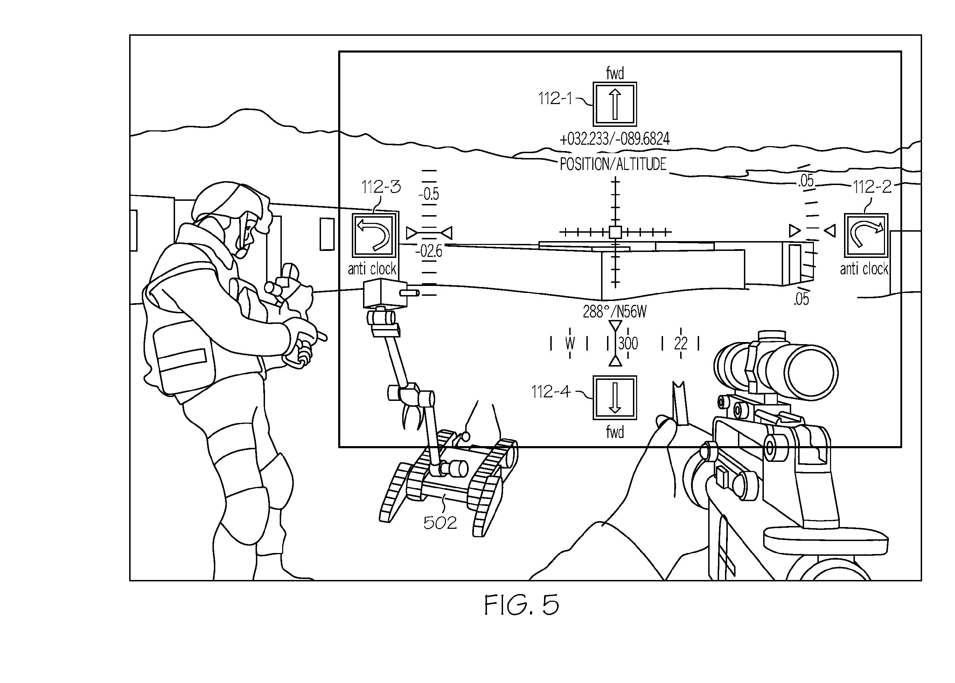

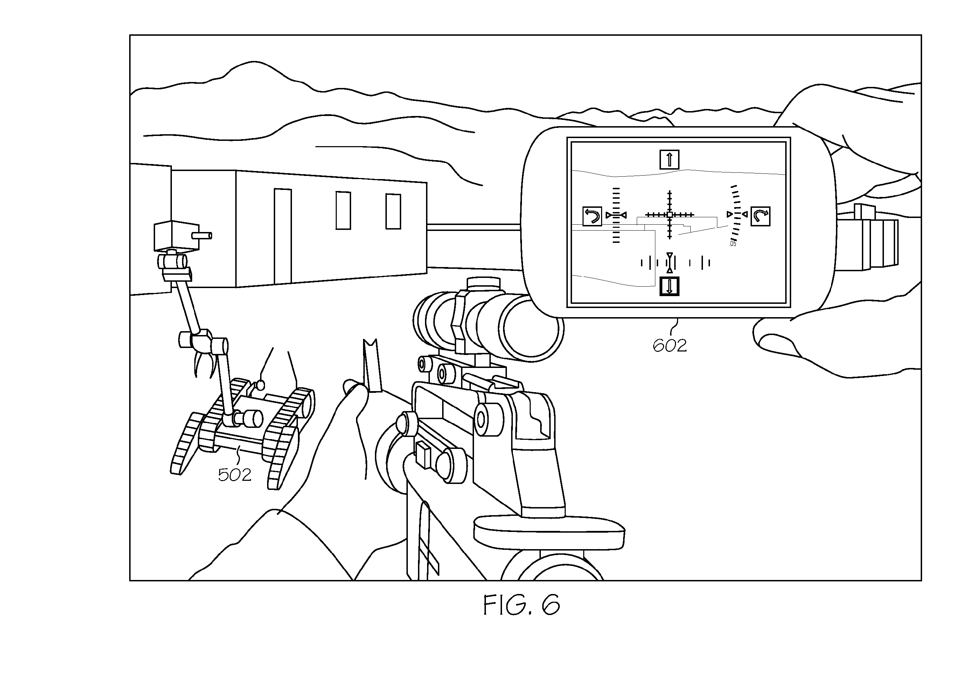

Another example of a multiple degree-of-freedom system is a robotic system, such as an unmanned land or aerial vehicle. One particular example of an unmanned land vehicle is depicted in FIGS. 5 and 6. In these depicted embodiments, the unmanned land vehicle is a military-related ordinance vehicle 502 that is configured to not only be controllably moved over the ground, but to also target and/or fire upon enemy combatants or enemy assets. To this end, the visual user interface 102 may be implemented into an HUD, as illustrated in FIG. 5, or it may be implemented into a camera-enabled augmented reality interface on a mobile device 602 that is dimensioned to be held in single hand of the user 110, as illustrated in FIG. 5.

No matter how the visual user interface 102 is implemented with the robotic system 502, the visual stimuli 112 displayed thereon may include more than just the vehicle directional command stimuli 112 depicted in FIGS. 5 and 6. Indeed, the visual user interface 102 could be configured to display visual stimuli that may be used to specify waypoints on a 2-dimensional or 3-dimenasional map. Moreover, the system controller 108 is implemented to wirelessly transmit signals to, and receive signals from, the robotic system.

The systems and methods described herein provide a human-computer interface paradigm that applies flexibly across system and task contexts, including a hands-free paradigm that may be implemented with relatively complex, multiple degree-of-freedom systems and devices.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. An apparatus for controlling a multiple degree-of-freedom system, comprising:
a user interface configured to generate a plurality of stimuli to a user;
a plurality of bioelectric sensors configured to obtain and supply a plurality of steady state visual evoked response potential (SSVEP) signals from the user when the user is receiving the stimuli;
a processor coupled to receive the plurality of SSVEP signals from the bioelectric sensors and configured, upon receipt thereof, to determine a system command and supply a system command signal representative thereof; and
a system controller coupled to receive the command signal and configured, upon receipt thereof, to generate a plurality of component commands that cause the multiple degree-of-freedom system to implement the system command.

2. The apparatus of Claim 1, wherein:
the stimuli are visual stimuli;
the user has a physical visual system; and
the processor implements a dynamic model of the physical visual system of the user as a communication channel, the dynamic model representative of the dynamic behavior of the response of the physical visual system to the visual stimuli.

3. The apparatus of Claim 2, wherein:
the dynamic model generates a model-based response to the visual stimuli; and
the processor implements a model-based classifier, the model-based classifier configured to determine the system command in response to model-based response.

4. The apparatus of Claim 2, wherein:
the user interface is configured to display the plurality of visual stimuli in accordance with a flickering pattern; and
the flickering pattern is based on the dynamic model.

5. The apparatus of Claim 1, wherein the user interface is further configured to display images that are at least representative of a physical environment in which the multiple degree-of-freedom system is disposed.

6. A method for controlling a multiple degree-of-freedom system, comprising:
displaying, on a visual interface, a plurality of visual stimuli to a user;
obtaining a plurality of steady state visual evoked response potential (SSVEP) signals from the user when the user is viewing the visual interface;
processing the plurality of SSVEP signals to generate a system command; and
generating a plurality of component commands based on the system command, the plurality of components commands causing the multiple degree-of-freedom system to implement the system command.

7. The method of Claim 6, further comprising:
implementing a dynamic model of a physical visual system of the user as a communication channel, the dynamic model representative of the dynamic behavior of the response of the physical visual system to the stimuli.

8. The method of Claim 7, further comprising:
generating a model-based response to the visual stimuli using the dynamic model;
and
implementing a model-based classifier to determine the system command in response to model-based response.

9. The method of Claim 7, further comprising:
displaying the plurality of visual stimuli in accordance with a flickering pattern that is based on the dynamic model.
